# FASCICULE DE BREVET EUROPEEN

(11) **EP 1 781 214 B1**
(45) Date de publication et mention de la délivrance du brevet: **03.06.2020**
(21) Numéro de dépôt: 04786283.4
(22) Date de dépôt: 09.08.2004
(51) Int. Cl.: A61F 2/32

(54) **SYSTÈME D'IMPLANTS À TRIPLE ARTICULATION DU TYPE SPHÉRIQUE POUR MEMBRE SUPÉRIEUR OU INFÉRIEUR**
DREIFACHGELENK-IMPLANTATIONSSYSTEM VOM KUGELTYP FÜR OBERE UND UNTERE GLIEDMASSEN
BALL-TYPE TRIPLE-JOINT IMPLANT SYSTEM FOR UPPER OR LOWER LIMBS

(43) Date de publication de la demande: 09.05.2007
(73) Titulaire: Ortho - I.D. Sarl, 69500 Bron (FR); Bonnard, Olivier, 69330 Meyzieu (FR); Bauchu, Philippe, 69002 Lyon (FR); Cypres, Alain, 42190 St Nizier sous Charlieu (FR); Fiquet, Arnaud, 69300 Caluire (FR); Girardin, Philippe, 42600 Lezigneux (FR); Noyer, Daniel, 38200 Luzinay (FR)
(72) Inventeur: BONNARD, Olivier, F-69330 Meyzieu (FR); BAUCHU, Philippe, F-69002 Lyon (FR); CYPRES, Alain, F-42190 ST. NIZIER SOUS CHARLIEU (FR); FIQUET, Arnaud, F-69300 Caluire (FR); GIRARDIN, Philippe, F-42600 Lezigneux (FR); NOYER, Daniel, F-38200 Luzinay (FR); MOULIN, Jean-Pierre, F- 69006 LYON (FR); ROY, Christophe, F-69001 Lyon (FR)
(74) Mandataire: Brédeville, Odile Marie
(86) Numéro de dépôt international: PCT/FR2004/002110
(87) Numéro de publication internationale: WO 2006/027422

(56) Documents cités:
- DE-A1- 4 428 407
- FR-A- 2 662 930
- FR-A- 2 692 776
- FR-A- 2 807 315
- FR-A- 2 850 861
- US-A- 4 955 916
- US-A- 4 960 427

## Description

### I/ Domaine technique et état de l'art :

Les dispositifs connus à ce jour pour la réalisation d'un palier articulaire pour remplacer les articulations du membre supérieur ou inférieur de type sphérique sont de quatre types :
1/ Des cupules cimentées dans la cavité osseuse, destinées à recevoir une bille de prothèse sont décrites dans les documents FR 2610515 et FR 2618065.
2/ Des cupules mobiles simples dites « intermédiaires » s'articulant sur une bille de prothèse et dans la cavité osseuse sur le reste de cartilage car non fixées dans ladite cavité osseuse sont décrites dans le document FR 2558721.
3/ Des cupules métalliques dites «sans ciment » introduites en force dans la cavité osseuse préparée grâce à une outil de forme, et munies d'un insert articulaire fixe destiné à recevoir une bille de prothèse sont décrites dans les documents FR 2703904 et FR 2652498.
4/ Des cupules métalliques dites sans ciment similaires pour l'extérieur au type 3 mais présentant une face interne concave polie pour recevoir une cupule mobile s'articulant à la fois dans cette concavité et sur une bille de prothèse sont décrites dans la demande de brevet FR 2662930.

Ce dernier type d'implants bien que séduisant par son principe est assez décevant à moyen terme car, du fait de la rétention de la bille dans la cupule mobile, celle ci présente une usure avec création importante de débris pouvant conduire à une reprise chirurgicale ou même à la luxation nécessitant le remplacement des implants.

Les documents US 4960427 et DE 4428407 décrivent un système pour remplacement des articulations osseuses de géométrie sensiblement sphérique comprenant une coquille d'ancrage, une cupule et une prothèse comprenant une bille et un col.

### II/ Principes et avantages attendus de l'invention :

Il est important et économique d'offrir aux patients des implants pour remplacement des articulations des membres supérieurs ou inférieurs stables, exempts de luxation même en cas de grande mobilité, particulièrement en circumduction, et dont l'usure est minimale afin de réduire les remplacements de prothèses sur une population agée dont l'espérance de vie augmente, mais pour laquelle les interventions de reprise sont lourdes tant en mortalité qu'en morbidité. La triple articulation objet de l'invention se produit - d'une part, pour la première articulation, entre la face externe d'une cupule mobile en matériau plastique bio-compatible et entre l'intérieur d'une coquille d'ancrage osseux en matériau rigide bio-compatible présentant une face à concavité sensiblement hémisphérique polie exempte d'aspérités, de cavités ou de reliefs pouvant user la dite cupule mobile ou entraver son libre débattement, lorsque cette dernière est sollicitée en déplacement rotatoire par le col de la prothèse, - d'autre part, pour la deuxième articulation, entre la surface articulaire polie de la bille d'une prothèse et entre la face interne de la cupule mobile dont la superficie est de préférence supérieure à une demi sphère de diamètre similaire à celui de ladite bille, et également d'autre part, pour la troisième articulation, entre le col de ladite prothèse, caractérisé en ce qu'il est lisse sans aspérité ni encoche et de préférence légèrement évasé, pour permettre le mouvement de circumduction articulaire physiologique à frottement doux exempt d'usure, et entre le chanfrein situé à l'ouverture de la cupule mobile, caractérisé en ce que cette ouverture se rétrécit vers le centre de ladite cupule articulaire mobile en présentant un diamètre intérieur sensiblement égal ou inférieur à celui de la bille, et dont l'évasement est conçu pour s'adapter parfaitement avec celui du col évasé de la prothèse, dans toutes les positions respectives des pièces mobiles lorsque le col vient en contact avec la cupule mobile et l'entraîne dans sa course en cas de débattement de grande amplitude.

Il s'agit donc d'un dispositif qui, associant la fonctionnalité de systèmes par ailleurs déjà décrits, apporte une réponse aux problèmes d'usure et de détérioration rapide et permet donc de traiter avec succès, sans usure, sans luxation, donc pour plus longtemps et avec une grande amplitude de mouvements, par sa triple articulation, l'ensemble des pathologies articulaires.

Les caractéristiques les plus importantes du système d'après l'invention sont réitérées dans les revendications.

### III/ Description des modes de réalisation et dessins :

### EXPLICATION DES FIGURES:

D'autres avantages et aspects de l'invention ressortiront à la lecture de la description suivante faite à titre d'exemple et en référence aux dessins annexés dans lesquels :

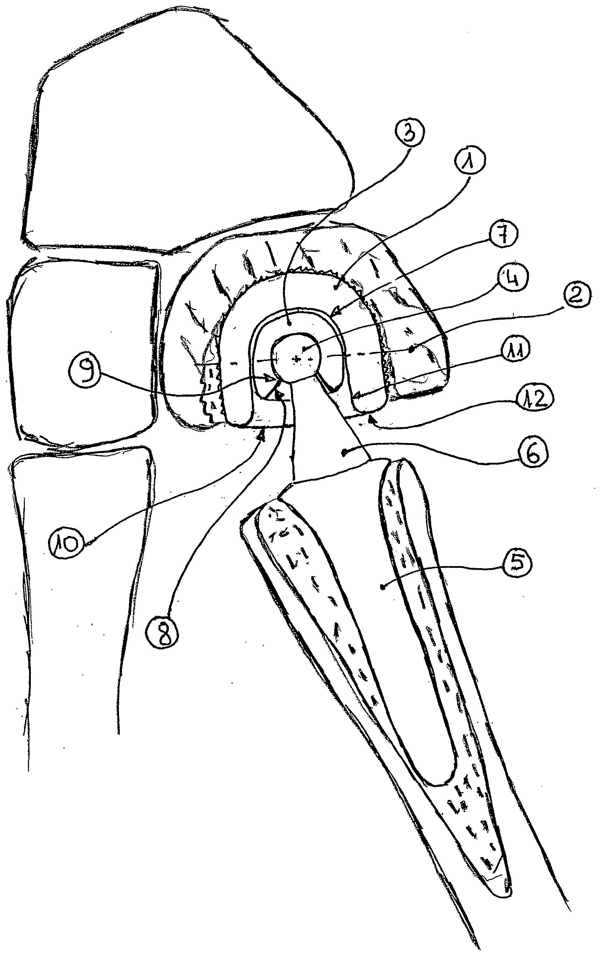

La figure 1 est une vue en perspective du système d'articulation osseuse (1) comprenant trois composants articulaires, à savoir une coquille d'ancrage osseux (1) et une cupule articulaire dans sa configuration mobile (3) glissant d'une part sur la tête (4) d'une prothèse fémorale (5) et d'autre part dans la cavité lisse (7) en forme de portion de sphère de la coquille d'ancrage osseux (1)

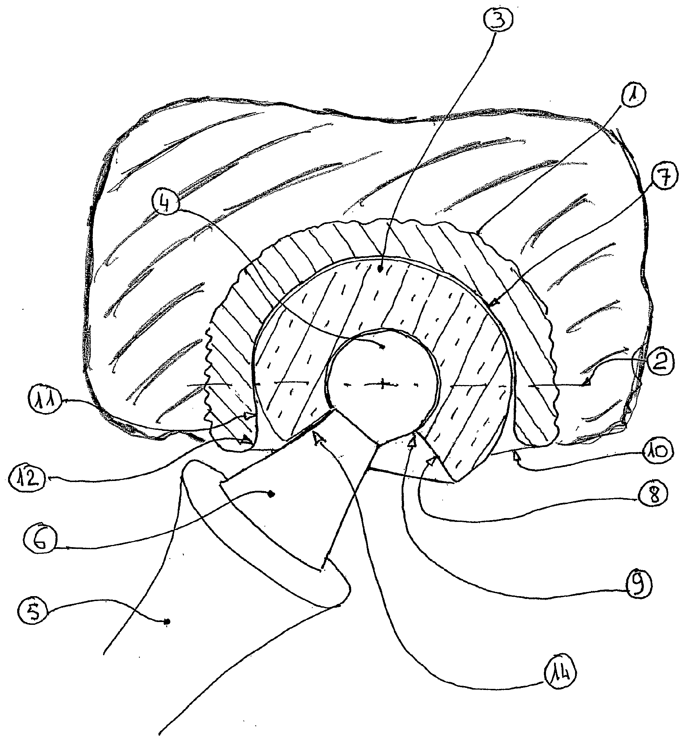

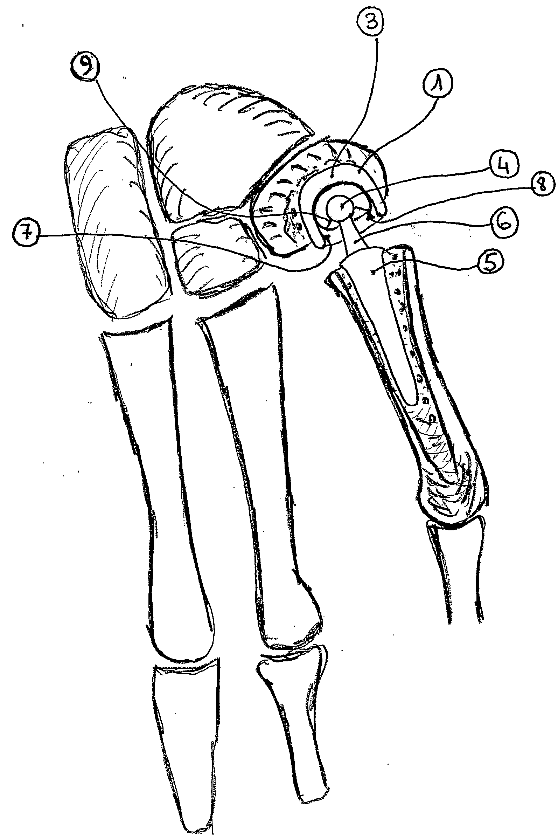

La figure 2 est une vue de détail montrant la troisième articulation avec les caractéristiques revendiquées pour le col (6) et pour le chanfrein interne (8) de la cupule mobile (3)

L'invention n'est pas limitée aux formes de réalisation représentées dans les dessins annexés.

### DESCRIPTION:

Système pour remplacement des articulations osseuses de géométrie sensiblement sphérique des membres supérieurs et inférieurs, réalisé en matériaux bio-compatibles, caractérisé en ce qu'il comprend trois composants articulaires et une triple articulation avec trois surfaces de contact de glissement concourant au déplacements, à savoir :
- Une coquille d'ancrage osseux (1) en matériau rigide bio-compatible présentant à sa face interne concave une surface articulaire lisse (7), sensiblement hémisphérique polie exempte d'aspérités, de cavités ou de reliefs pouvant user la cupule mobile (3) ou entraver son libre débattement, lorsque cette dernière est sollicitée en déplacement angulaire ou rotatoire par le col (6) de la prothèse (5), caractérisée en ce que son ouverture (10) est située à une distance comprise entre
   de let 6mm au delà de l'équateur (2) de ladite coquille d'ancrage osseux (1) de manière à réaliser une jupe de guidage (11) et présente un bord arrondi (12) avec un rayon compris entre 1 et 5mm, afin d'éviter toute abrasion de la cupule mobile (3) lors de son introduction per-opératoire dans ladite coquille d'ancrage osseux (1).
- Une prothèse (5) comportant, à sa partie opposée à la partie d'ancrage diaphysaire, une bille (4) polie et un col (6) caractérisé en ce qu'il et lisse sans aspérité ni encoche et de préférence légèrement évasé, pour permettre le mouvement de circumduction articulaire physiologique à frottement doux exempt d'usure.
- Une cupule articulaire mobile (3), glissant d'une part dans la cavité lisse (7) en forme de portion de sphère de la coquille d'ancrage osseux (1) et d'autre part sur la bille (4) et le col (6) d'une prothèse (5) à ancrage diaphysaire, caractérisée en ce que son ouverture (8) se rétrécit vers le centre de ladite cupule articulaire mobile (3) en présentant un diamètre intérieur (9) sensiblement égal ou inférieur à celui de la bille (4), et dont l'évasement est conçu pour s'adapter parfaitement avec le col (6) évasé de la prothèse (5), dans toutes les positions respectives des pièces mobiles.

Ledit système d'articulation permettant grâce à ses caractéristiques particulières d'assurer avec succès, même en cas de révision ou de mauvaise qualité osseuse, un remplacement durable et stable permettant des mouvements de grande amplitude sans luxation ni usure pour les articulations de type sphériques du membre supérieur ou inférieur.

Plus particulièrement :
- Le système pour remplacement des articulations osseuses de géométrie sensiblement sphérique à triple articulation dont la troisième articulation se situe entre le col (6) de la prothèse (5), lisse et sans aspérité ni encoche et de préférence légèrement évasé et entre le chanfrein situé à l'ouverture de la cupule mobile (3) est caractérisé en ce que son évasement est compris entre 1 et 15° de préférence 6° pour permettre le mouvement de circumduction articulaire physiologique à frottement doux exempt d'usure et pour s'adapter parfaitement avec celui du col (6) évasé de la prothèse (5), dans toutes les positions respectives des pièces mobiles.
- Le système pour remplacement des articulations osseuses de géométrie sensiblement sphérique à triple articulation dont la troisième articulation est située entre le col (6) de la prothèse (5), lisse sans aspérité ni encoche et légèrement évasé de préférence à 6° pour permettre le mouvement de circumduction articulaire physiologique et entre le chanfrein situé à l'ouverture de la cupule mobile (3), est caractérisé en ce que l'évasement est déterminé par un angle correspondant au débattement angulaire choisi, selon le degré de liberté de l'articulation à remplacer, augmenté de l'angle dudit col (6) afin de réaliser un contact (14) en alignement linéaire parfait des deux surfaces pour réaliser un frottement doux exempt d'usure entre les pièces mobiles, dans toutes leurs positions respectives.
- Le système pour remplacement des articulations osseuses de géométrie sensiblement sphérique à triple articulation dont la deuxième articulation se situe entre la surface articulaire polie de la bille (4) d'une prothèse (5) et entre la face interne de la cupule mobile (3) est caractérisé en ce que la superficie de ladite face interne est de préférence supérieure à une demi sphère de diamètre similaire à celui de la bille (4), et que son ouverture (8) rétrécie vers le centre de ladite cupule articulaire mobile (3) présente un diamètre intérieur (9) inférieur de 0,5 à 4mm, de préférence de 1/10^{ème} à celui de la bille (4) afin de renforcer la stabilité de ladite bille (4) dans la cupule mobile (3).

## Revendications

1. Système pour remplacement des articulations osseuses de géométrie sensiblement sphérique, réalisé en matériaux bio-compatibles, comprenant trois composants articulaires et une triple articulation avec six surfaces de contact de glissement concourant aux déplacements, à savoir:
- une coquille d'ancrage osseux (1) en matériau rigide bio-compatible présentant à sa face interne concave une surface articulaire lisse (7) sensiblement hémisphérique polie qui est exempte d'aspérités, de cavités ou de reliefs pouvant user une cupule articulaire mobile (3) ou entraver son libre débattement, lorsque cette dernière est sollicitée en déplacement rotatoire par le col (6) d'une prothèse à ancrage diaphysaire (5) ;
- la prothèse (5) comportant à la partie opposée à sa partie d'ancrage diaphysaire une bille (4) polie et un col (6) qui est lisse sans aspérité ni encoche et de préférence légèrement évasé, pour permettre le mouvement de circumduction articulaire physiologique à frottement doux exempt d'usure ;
- la cupule articulaire mobile (3), glissant d'une part dans la cavité lisse (7) en forme de portion de sphère de la coquille d'ancrage osseux (1) et d'autre part sur la bille (4) et le col (6) de la prothèse (5), une ouverture (8) de ladite cupule mobile (3) se rétrécissant vers le centre de ladite cupule articulaire mobile (3) et dont l'évasement est conçu pour s'adapter parfaitement avec le col (6) évasé de la prothèse (5), dans toutes les positions respectives des pièces mobiles, et dont la première articulation se situe entre la face externe de la cupule articulaire mobile (3) réalisée en matériau bio-compatible de préférence plastique, et entre l'intérieur de la coquille d'ancrage osseux (1) en matériau rigide bio-compatible présentant une face (7) à concavité sensiblement hémisphérique polie exempte d'aspérités, de cavités ou de reliefs pouvant user la dite cupule mobile (3) ou entraver son libre débattement, **caractérisé en ce que** l'ouverture (10) de ladite face (7) à concavité hémisphérique est située à une distance comprise entre de 1 et 6mm au delà de l'équateur (2) de ladite coquille d'ancrage osseux (1) de manière à réaliser une jupe de guidage (11) et présente un bord arrondi (12) avec un rayon compris entre 1 et 5mm, afin d'éviter toute abrasion de la cupule mobile (3) lors de son introduction per-opératoire dans ladite coquille d'ancrage osseux (1).

2. Système pour remplacement des articulations osseuses de géométrie sensiblement sphérique à triple articulation selon la revendication 1 **caractérisé en ce que** la troisième articulation se situe entre le col (6) de la prothèse (5), lisse sans aspérité ni encoche et de préférence légèrement évasé, et entre le chanfrein situé à l'ouverture de la cupule mobile (3), son évasement étant compris entre 1 et 15° de préférence 6° pour permettre le mouvement de circumduction articulaire physiologique à frottement doux exempt d'usure et pour s'adapter parfaitement avec celui du col (6) évasé de la prothèse (5), dans toutes les positions respectives des pièces mobiles.

3. Système pour remplacement des articulations osseuses de géométrie sensiblement sphérique à triple articulation selon au moins une des revendications 1 et 2 **caractérisé en ce que** la troisième articulation est située entre le col (6) de la prothèse (5), lisse sans aspérité ni encoche et légèrement évasé de préférence à 6° pour permettre le mouvement de circumduction articulaire physiologique et entre le chanfrein situé à l'ouverture de la cupule mobile (3), l'évasement étant déterminé par un angle correspondant au débattement angulaire choisi, selon le degré de liberté de l'articulation à remplacer, augmenté de l'angle dudit col (6) afin de réaliser un contact (14) en alignement linéaire parfait des deux surfaces pour réaliser un frottement doux exempt d'usure entre les pièces mobiles, dans toutes leurs positions respectives.

4. Système pour remplacement des articulations osseuses de géométrie sensiblement sphérique à triple articulation selon l'une quelconque des revendications 1 à 3 **caractérisé en ce que** la deuxième articulation se situe entre la surface articulaire polie de la bille (4) d'une prothèse (5) et entre la face interne de la cupule mobile (3) la superficie de ladite face interne étant de préférence supérieure à une demi sphère de diamètre similaire à celui de la bille (4), et que son ouverture (8) rétrécie vers le centre de ladite cupule articulaire mobile (3) présente un diamètre intérieur (9) inférieur de 0,5 à 4mm, de préférence de 1/10ème à celui de la bille (4) afin de renforcer la stabilité de ladite bille (4) dans la cupule mobile (3).

## Patentansprüche

1. System zum Ersetzen der Knochengelenke mit etwa kugeliger Geometrie, hergestellt aus biologisch verträglichen Materialien, umfassend drei Gelenkkomponenten und ein Dreifachgelenk mit sechs gleitenden Kontaktflächen, die zu den Verlagerungen beitragen, nämlich:
- eine Knochenverankerungsschale (1) aus festem biologisch verträglichem Material, aufweisend auf ihrer konkaven inneren Fläche eine etwa halbkugelige glatte polierte Gelenkoberfläche (7) ohne Unebenheiten, Hohlräume oder Reliefs, die eine bewegliche Gelenkpfanne (3) verschleißen oder ihren freien Ausschlag beeinträchtigen könnten, wenn diese in Rotationsverlagerung vom Hals (6) der Prothese mit diaphysärer Verankerung (5) beansprucht wird;
- wobei die Prothese (5) an dem Teil, das ihrem Teil diaphysärer Verankerung zugewandt ist, eine polierte Kugel (4) und einen Hals (6) aufweist, der ohne Unebenheit oder Kerbe glatt und vorzugsweise leicht erweitert ist, um die physiologische Kreisbewegung des Gelenks mit sanfter Reibung verschleißfrei zu erlauben;
- die bewegliche Gelenkpfanne (3), die zum einen in dem glatten Hohlraum (7) in Kugelabschnittform der Knochenverankerungsschale (1) und zum anderen auf der Kugel (4) und dem Hals (6) der Prothese (5) mit diaphysärer Verankerung gleitet, wobei sich eine Öffnung (8) der beweglichen Pfanne (3) zur Mitte der beweglichen Gelenkpfanne (3) verengt und deren Erweiterung ausgebildet ist, um sich perfekt an den erweiterten Hals (6) der Prothese (5) in allen jeweiligen Positionen der beweglichen Teile anzupassen,
und wobei sich das erste Gelenk zwischen der äußeren Fläche der beweglichen Gelenkpfanne (3), hergestellt aus vorzugsweise biologisch verträglichem Kunststoffmaterial, und zwischen dem Inneren der Knochenverankerungsschale (1) aus festem biologisch verträglichen Material befindet, aufweisend eine Fläche (7) mit etwa halbkugeliger polierter Konkavität ohne Unebenheiten, Hohlräume oder Reliefs, die eine bewegliche Pfanne (3) verschleißen oder ihren freien Ausschlag beeinträchtigen könnten, **dadurch gekennzeichnet, dass** sich die Öffnung (10) der Fläche (7) mit halbkugeliger Konkavität in einem Abstand zwischen 1 und 6 mm inklusive jenseits des Äquators (2) der Knochenverankerungsschale (1) befindet, so dass ein Führungsmantel (11) gebildet wird, und einen abgerundeten Rand (12) mit einem Radius zwischen 1 und 5 mm inklusive aufweist, um jedweden Abrieb der beweglichen Pfanne (3) bei ihrem Einsetzen während der Operation in die Knochenverankerungsschale (1) zu vermeiden.

2. System zum Ersetzen der Knochengelenke mit etwa kugeliger Geometrie mit Dreifachgelenk nach Anspruch 1, **dadurch gekennzeichnet, dass** sich das dritte Gelenk zwischen dem ohne Unebenheit oder Kerbe glatten und vorzugsweise leicht erweiterten Hals (6) der Prothese (5) und zwischen der Schräge an der Öffnung der beweglichen Pfanne (3) befindet, wobei seine Erweiterung zwischen 1 und 15° inklusive, vorzugsweise 6°, beträgt, um die physiologische Kreisbewegung des Gelenks mit sanfter Reibung verschleißfrei zu erlauben und um sich perfekt an die des erweiterten Halses (6) der Prothese (5) in allen jeweiligen Positionen der beweglichen Teile anzupassen.

3. System zum Ersetzen der Knochengelenke mit etwa kugeliger Geometrie mit Dreifachgelenk nach mindestens einem der Ansprüche 1 und 2, **dadurch gekennzeichnet, dass** sich das dritte Gelenk zwischen dem ohne Unebenheit oder Kerbe glatten und vorzugsweise leicht um 6° erweiterten Hals (6) der Prothese (5), um die physiologische Kreisbewegung des Gelenks zu erlauben, und zwischen der Schräge an der Öffnung der beweglichen Pfanne (3) befindet, wobei die Erweiterung von einen Winkel bestimmt ist, der dem gewählten Ausschlag gemäß dem Freiheitsgrad des zu ersetzenden Gelenks plus dem Winkel des Halses (6) entspricht, um einen perfekt linear ausgerichteten Kontakt (14) der zwei Oberflächen herzustellen, um eine sanfte Reibung verschleißfrei zwischen den beweglichen Teilen in allen jeweiligen Positionen herzustellen.

4. System zum Ersetzen der Knochengelenke mit etwa kugeliger Geometrie mit Dreifachgelenk nach mindestens einem der Ansprüche 1 bis 3, **dadurch gekennzeichnet, dass** sich das zweite Gelenk zwischen der polierten Gelenkoberfläche der Kugel (4) einer Prothese (5) und zwischen der inneren Fläche der beweglichen Pfanne (3) befindet, wobei die Oberfläche der inneren Fläche vorzugsweise größer als eine halbe Kugel ähnlichen Durchmessers wie der der Kugel (4) ist, und dass ihre zur Mitte der beweglichen Gelenkpfanne (3) verengte Öffnung (8) einen Innendurchmesser (9) aufweist, der um 0,5 bis 4 mm, vorzugweise um 1/10 als der der Kugel (4), kleiner ist, um die Stabilität der Kugel (4) in der beweglichen Pfanne (3) zu verstärken.

## Claims

1. A system for replacing bone joints of substantially spherical geometry, made of biocompatible materials, comprising three articular components and a triple joint with six sliding contact surfaces for the displacements, namely:
- A bone-anchoring shell (1) made of rigid biocompatible material and having, on its concave inner face, a smooth and substantially hemispherical articular surface (7) which is polished and has no bumps, cavities or reliefs that could cause wear of an articular mobile cup (3) or could impede its free clearance when the latter is stressed in rotary displacement by the neck (6) of a prosthesis (5) for diaphyseal anchoring;
- the prosthesis (5) comprising, at the part remote from its diaphyseal anchoring part, a polished ball (4) and a neck (6) which is smooth, without bumps or notches, and is preferably slightly flared, in order to permit the joint physiological circumduction movement with low friction and without wear;
- the mobile articular cup (3) sliding, on the one hand, in the smooth cavity (7), shaped as a portion of a sphere of the bone-anchoring shell (1) and, on the other hand, on the ball (4) and the neck (6) of the prosthesis (5),
an opening (8) of said mobile cup (3) narrowing toward the center of said mobile articular cup (3), the flared part of which being designed to adapt perfectly to the flared neck (6) of the prosthesis (5), in all the respective positions of the mobile components,
the first joint of which is situated between the outer face of the mobile articular cup (3) made of preferably plastic biocompatible material, and between the inside of the bone-anchoring shell (1) made of rigid biocompatible material having a face (7) with a substantially hemispherical concavity polished and free of bumps, cavities or reliefs that could wear said mobile cup (3) or impede its free clearance, **characterized in that** the opening (10) of said face (7) of hemispherical concavity is situated at a distance of between 1 and 6 mm beyond the equator (2) of said bone-anchoring shell (1) in such a way as to form a guide skirt (11), and shows a rounded edge (12) with a radius of between 1 and 5 mm, in order to avoid any abrasion of the mobile cup (3) during its introduction into said bone-anchoring shell (1) during surgery.

2. The system for replacing bone joints of substantially spherical geometry with triple joint as claimed in claim 1, **characterized in that** the third joint is situated between the neck (6) of the prosthesis (5), smooth and without bumps or notches, and preferably slightly flared, and between the bevel situated at the opening of the mobile cup (3), the flare of which being between 1 and 15°, preferably 6°, in order to permit the joint physiological circumduction movement with low friction and without wear, and to adapt perfectly to that of the flared neck (6) of the prosthesis (5), in all the respective positions of the mobile components.

3. The system for replacing bone joints of substantially spherical geometry with triple joint as claimed in at least one of claims 1 and 2, **characterized in that** the third joint is situated between the neck (6) of the prosthesis (5), smooth and without bumps or notches, and slightly flared, preferably at 6°, in order to permit the joint physiological circumduction movement, and between the bevel situated at the opening of the mobile cup (3), the flare being defined by an angle corresponding to the angular clearance chosen, depending on the degree of freedom of the joint to be replaced, augmented by the angle of said neck (6) in order to form a contact (14) in perfect linear alignment of the two surfaces, so as to produce low friction, without wear, between the mobile components, in all their respective positions.

4. The system for replacing bone joints of substantially spherical geometry with triple joint as claimed in any one of claims 1 to 3, **characterized in that** the second joint is situated between the polished articular surface of the ball (4) of a prosthesis (5) and between the inner face of the mobile cup (3), the surface area of said inner face being preferably greater than a hemisphere of diameter similar to that of the ball (4), and **in that** its opening (8) narrowing toward the center of said mobile articular cup (3) has an internal diameter (9) of less than 0.5 to 4 mm, preferably 1/10th that of the ball (4), in order to reinforce the stability of said ball (4) in the mobile cup (3) .
